# EUROPEAN PATENT APPLICATION

(11) **EP 1 426 368 A1**
(43) Date of publication of application: **09.06.2004**
(21) Application number: 00940429.4
(22) Date of filing: 05.07.2000
(51) Int. Cl.: C07D 311/72, C11B 7/00

(54) **METHOD FOR EXTRACTING AND PURIFYING NATURAL TOCOPHEROLS AND STEROLS BY ESTERIFICATION WITH TRIMETHYL PROPANE**

(30) Priority: 28.06.2000 ES 200001604
(71) Applicant: Vitae - Caps, S.A., 45614 Talavera de la Reina (ES)
(72) Inventor: ELOY MURO, Andrés, E-45614 Talavera de la Reina (ES)
(74) Representative: Riera Blanco, Juan Carlos
(86) International application number: PCT/ES2000/000237
(87) International publication number: WO 2002/000640

(57) **Abstract**

Procedure for extracting and purifying natural-occurring tocopherols and sterols through sterification with propane trimethylol, consisting of specially catalyzed sterification aimed to convert fatty acids from the deodorization distillation into esters characterized by high molecular weight and little volatility, in order that the residue shall be retained in a selective distillation. The alcohol for fatty acid sterification is propane trimethylol, with hypophosphorus acid being used as the catalyzer. In the dilution, methanol is used in a 1:7 proportion. Filtration or spin is made at -30°C. Molecular distillation is carried out in order to split esters from fatty acid, propane trimethylol, tocopherols, sterols and hydrocarbons. Molecular distillation is also intended to purify tocopherols.

## Description

### SUBJECT OF THE INVENTION

The present Specification refers to an application for Patent of Invention relating to the extraction and purification process of natural-occurring tocopherols and sterols through sterification with propane trimethylol (PTM), with the obvious aim of which being to obtain tocopherols and sterols from natural sources, mainly from concentrates obtained by deodorization of vegetable oils.

Briefly, the process being proposed as an invention basically consists of a specially catalyzed sterification intended to convert free fatty acids being present in the deodorization distillation into esters with a high molecular weight and little volatility, so that they shall be retained in the residue of a selective distillation.

### SCOPE

This invention has potential applications in the fields of chemical and pharmaceutical industries.

### BACKGROUND

Vegetal oils, such as sunflower, soy, rapeseed and corn oils, are known to be valuable natural sources of tocopherols and sterols. Also, these oils contain, aside form tocopherols and sterols, fatty acids, glycerides (mono-, di- and tri-glicerydes), non-glyceride esters, hydrocarbons, etc.

Obtaining natural tocopherols and sterols from these natural sources is of great industrial concern. There exist several previous reports on processes aimed to obtain tocopherols and sterols from natural sources; however, comprehensive itemized research of these reports makes us to conclude that these processes should be discarded because of their low industrial efficiency, low concentrations of both tocopherols and sterols, by-products release and, generally speaking, because they all can be deemed as being unprofitable for their industrial exploitation.

Tocopherol compounds are contained in many vegetable and animal oils. These compounds are also referred to as vitamin E.

The term vitamin E refers to the physiological effect of these compounds when contained in foods and drinks.

Eight different natural-occurring substances are known to have vitamin E effect, which are derivatives from 6-chromatol and are categorized into two distinct compound groups.

Compounds in the first group come from tocol and carry an isoprenoid side chain saturated with 16 carbon atoms. Alpha-, beta-, gamma- and delta-tocopherol belong all to this group, with the different compounds being differentiated by the methylation degree on the tocol benzene ring. Alpha-tocopherol has the strongest vitamin E biological effect and the highest industrial and economic significance, this being, on the other hand, the prevailing tocopherol form found in human beings' and animal tissues.

The second substance group having vitamin E effect is made up by the derivatives from tocotrienol, and these are differentiated from the other tocopherol homologous by the unsaturated isopreinod 16-carbon atom side chain they present.

Natural-occurring tocopherols also possess vitamin E effect and are isolated with the tocopherol saturated homologous from their natural sources while obtaining vitamin E. The term "tocopherol" in the present application also embraces these tocopherol homologous (i.e., substances having vitamin E effect).

Tocopherols have industrial applications because of their oxidation- inhibiting properties in foods and drinks and in the cosmetic and pharmaceutical fields, as well as for additives in natural oil-based paints.

In the present application, the term "sterol" refers to sterols, also called sterines. Hence, it should be noted that the terms "sterol" and "sterine" are used in the present specification as being equivalent.

Sterols are one-valence secondary steroid alcohols, having an amount of carbon atoms ranging between 27 and 30, presenting the gonane basic structure, with the gonane carbon atom 3 carrying the hydroxyl group.

Structural differences between individual sterols found up to date in nature are based on the presence of double bounds within the annular system, the incorporation of substitutes in preferential points and the side chain construction, which is linked to the gonane carbon atom 17.

The most prominent representative for sterols is cholesterine, which is present, either free or sterificated, in animal organs and body flows, most notably in the brain, spinal cord and adrenals, as well as in cod-liver oil and lanolin.

Cholesterine is classified into the so-called zoosterines, a term used to refer to sterines contained in animal fat, while vegetable sterines are called phytosterines.

Most prominent representatives are eargosterine, stigmastearine, capesterine and sitosterine

Sterines or sterols are valuable raw materials for pharmaceutical product synthesis, particularly of steroid hormones, such as corticoids and gestagens. For instance, they can easily transform stigmaterine into progesterone.

Sources for obtaining tocopherol and sterol may be a variety of vegetable and animal substances, with the highest tocopherol concentrations occurring in vegetable oils, such as wheatseed, corn, soy, and palm oils.

Tocopherol is, however, found in other vegetable oils as well, such as safflower, peanut, cottonseed, sunflower, rapeseed, and palm oils.

Nevertheless, natural-occurring vegetable oils contain only tiny amounts of tocopherol while large concentrations are required for industrial applications. Of note, all impurities must be removed if vitamin E antioxidant effect and activity are to be reinforced.

Therefore, most important natural tocopherol sources are in no way vegetable oils themselves, but the distillates with water steam obtained by deodorizing vegetable and animal oils, which are referred to as deodorization distillates. In such a case, tocopherols are certainly obtained under a concentrated condition, but it should be borne in mind that they are still mixed with sterol, sterol esters, free fatty acids and glycerides.

The distillate from soy oil deodorization is particularly appealing and, for instance, reference to this process is made in the publication Fat Sci. Technol., 91, 1989, pages 39 through 41m, including a mention to the special adequacy of soy oil as a tocopherol source and a comparison of soy and rapeseed oil deodorization distillates

Soy deodorization distillate contains between 6% and 10%, approximately in total weight of mixed tocopherols and sterols in the same magnitude rank as seen in the preponderant volume in their ester form.

Tocopherols are widely used in food and pharmaceutical industries as antioxidants. They are obtained either through chemical synthesis processes or extraction from natural sources,

Because of the low concentration in which they are found in vegetable materials, isolation is mainly carried out from a by-product obtained from the oil deodorization process, with these distillates also containing fatty acids, glycerides, oils, sterols, oxidation products and other impurities to be removed.

For this purpose, a variety of physical-chemical processes, including sterification, saponification, extraction, molecular distillation, ionic exchange, absorption chromatography, sterol precipitation and crystallization, has been employed

Documentary or background research for the present invention report has been focused on the way of obtaining tocopherol from raw vegetable materials, with special emphasis on deodorized substances from soy and sunflower oils. The processes for obtaining tocopherol through chemical synthesis, alpha-tocopherol production from its homologous, and the processes related to the application of the compounds in cosmetic and food industries have not been addressed in the present documentary/background research.

It should be noted that most of the reports encountered in the research for the present application state the importance of splitting fatty acids; however, in general, they hardly ever emphasize strongly enough the importance of glyceride splitting. Therefore, it is to be stressed that glycerides are likely to remain in tocopherol concentration once fatty acid splitting has been completed.

One of the most important facts is the splitting of fatty acids by using a sterification process followed by distillation. Many reports in the existing literature refer to how this splitting can be performed, most often by sterification with alcohol, so that, as a result, fatty acid esters are produced, which, in turn, will be further split by distillation. Other reports state that the use of excessive amounts of alcohol may result in glyceride trans-sterification.

Some invention patent reports are included hereinafter in which distillation is used to split fatty acids.

### A. Distillation as a process for fatty acid splitting

Patent of Invention E52, 043,560, owned by ACEITES ALFONSO Y CIA, wherein from soy/sunflower oil two consecutive distillations are undertaken, with no complete separation of either sterols or fatty acids.

Patent of Invention W09700309 of Mr. DOMINGO SAURA LOPEZ, wherein soy and sunflower oil deodorization is performed. One or two previous distillations are made in order to remove most of fatty acids. Then the distillate undergoes treatment with a lipase by performing two consecutive distillations and, finally, a treatment with alcohol is made so that solid precipitates are removed by spin.

It should be stressed that with this process the full removal of fatty acids is virtually achieved, with glycerides and sterols remaining in a large proportion.

European Patent of Invention 333,472, in which the initial material is palm oil. A distillation to leave out most of fatty acids can be initially carried out, then performing treatment with alcohol so that sterification of the remaining fatty acids and glyceride trans-sterification are accomplished, with crystallization being the last stage in the molecular distillation process.

Sterol splitting is not specifically mentioned in the latter invention report, and it can be speculated that sterol splitting takes place in the crystallization stage.

Patent of Invention US 4,454,329, in which soy and sunflower oils are contemplated. Deodorization can be directly subject to a distillation process in order to split fatty acids. Then, sterification with alcohol to split the remaining fatty acids is made by carrying out distillation/fractionation for concentration. A further hydrogenation treatment enables one to split tri-glycerides through fractionation by dissolution.

In the latter Patent of Invention, splitting of the sterols accompanying tocopherols is not mentioned.

### B. Combined stages for splitting of fatty acids, glycerides and sterols

In the Patent of Invention KR9402715, the initial splitting of sterols by crystallization, removing fatty acids through methyl-sterification, and further molecular distillation are described.

Patent of Invention W09721697, in which soy oil deodorization is performed. Treatment with alcohol is made as well, so that fatty acids are sterificated and glycerols trans-sterificated, with removal of excessive amount of alcohol by distillation. A washing process intended to remove glycerol and fatty acid alkylesters is made by carrying out tocopherol and sterol splitting by crystallization.

Patent of Invention US5424457, in which soy oil deodorization is undertaken, as well as fatty acid sterification and glyceride trans-sterification. Then, removal of excessive amounts of alcohol and fatty acid esters by distillation is performed by carrying out sterol crystallization and molecular distillation, as well as mixture concentration.

Patent of Invention US5660691, in which deodorization is carried out from vegetable oils in general, such as rice oil, with sterification being made with alcohol and successive distillations.

Patent of Invention ES2130294, in which soy oil treatment is carried out by previous degassing in order to warm the residues to force fatty acid sterification together with sterols to yield sterolesters. Two-fraction distillation (i.e., the one containing tocopherol being either subject to sterification with methanol or added calcium hydroxide) is made, which results in ionic exchange in a basic resin that is exploited to split tocopherols.

### C. Sterol splitting

The two invention patent reports encountered for the present application deal exclusively with the process of splitting sterols from the tocopherol/sterol mixtures.

Patent of Invention GB1, 008,767 in which no kind of initial source type is specified. The source is saponified with soda. Then acidulation/ sterification is allowed and water is added, allowing time to crystallize.

Patent of Invention US3, 092,624, in which soy oil is treated by splitting its sterols by adding pipezarine and by crystallization.

### The reports quoted below refer to Patents of Invention found in the WPI database.

Patent of Invention CN1099754, relating to NATURAL VITAMIN E EXTRACTION FROM NON-SAPONIFICABLE VEGETAL FATS, a process including saponification, alcoholysis, alcohol washing, molecular distillation and refining.

Patent of Invention CN1228427, relating to SOY OIL DEODORIZED FOR NATURAL VITAMIN E EXTRACTION AND SPLITTING BY SUPERCRITIC EXTRACTION WITH CARBON DIOXIDE.

Patent of Invention DE19, 652,522, relating to A METHOD FOR OBTAINING TOCOPHEROL AND STEROL CONCENTRATIONS THROUGH FRACTIONAL DISTILLATION FOLLOWED BY MOLECULAR DISTILLATION, a process consisting of fractional distillation followed by molecular distillation.

Patent of Invention DE3, 126,110, relating to the PRODUCTION OF TOCOPHEROL CONCENTRATE BY SPLITTING ITS FATTY ACIDS, HYDROGENATION AND EXTRACTION WITH SOLVENTS.

Patent of Invention DE3, 424,614, relating to the ORGANIC COMPONENTS EXTRACTION BY TOCOPHEROL SOLUTE USING SUPERCRITIC SOLVENTS.

Patent of Invention EP0191132, relating to TOCOPHEROL PURIFICATION BY USING AOKOXI TOCOPHEROL BORATE M- ESTERS

Patent of Invention EP0316729, relating to TOCOPHEROL AND STEROL ENRICHMENT BY CALCIUM HYDROXIDE TREATMENT IN THE PRESENCE OF WATER AND WATER MIXED WITH ORGANIC AND INERT SOLVENTS.

Patent of Invention JP49085223, relating to OLEAGINOUS PLANTS FOR TOCOPHEROL PRODUCTION BY EXTRACTION WITH NON-IONIC SULFACTANTS.

Patent of Invention JP56012383, relating to TOCOPHEROL SPLITTING BY METHYL-STERIFICATION AND TREATMENT WITH BASIC RESINS.

Patent of Invention JP5705988, relating to VEGETABLE OIL CONDENSATES FOR OBTAINING TOCOPHEROL AND TOCOTRIENOL WITH MONOHYDROXILIC ALCOHOLS AND KETONES.

Patent of Invention JP57144281, relating to PREPARATION OF TOCOPHEROL CONCENTRATES BY POLYOL ADDITION.

Patent of Invention JP59212487, relating to TOCOPHEROL PURIFICATION BY DISSOLUTION IN ORGANIC SOLVENTS AND ABSORPTION WITH NON-POLAR POLYMERS.

Patent of Invention US4, 550,183, relating to ORGANIC SOURCES FOR TOCOPHEROLS PURIFICATION BY CONTACT AND NEUTRALIZATION WITH CAUSTIC METHANOL.

Patent of Invention US5, 371,245, relating to TOCOPHEROL FRACTION SPLITTING IN OILS BY SAPONIFICATION WITH METALLIC ZINC AND PRECIPITATION OF ZINC SALTS OF FATTY ACIDS

One can conclude from the above quotations that, at present, there is no recognition of processes for extracting and purifying natural-occurring tocopherols and sterols by sterification with propane trimethylol (PTM).

### DESCRIPTION OF THE INVENTION

The procedure for natural-occurring tocopherols and sterols extraction and purification by sterification with propane trimethylol (PTM) proposed in the present invention represents on its own an obvious novelty within its application field. It basically consists of a specially catalyzed sterification aimed to convert free fatty acids being present in the deodorization distillation into high molecular weight esters with low volatility, so that they shall be retained in a selective distillation.

Following numerous tests, the present invention puts forward fatty acid sterification (35 % in weight approximately) with PTM. The aforementioned alcohol with 6 carbon atoms, 3 hydroxyl groups (alcohol) placed in primary carbons and with a molecular weight of almost 135 behaves in a very rational manner, with sterification degrees achieved being higher than 95%, at a temperature range starting at 120°C (at the beginning of sterification) and increasing up to 190°C (at sterification completion).

The process takes place in vacuum (35-40 torr), and, as a result, the water resulting from sterification is continuously removed, with the reaction balance shifting towards sterification, hence preventing hydrolysis.

The catalyzer used in the present process is hypophosphorous acid in a 50% aqueous solution, which, thanks to its antioxidant properties, effectively protects the chormane nucleus of tocopherol molecule from oxidation.

Reaction, with a temperature gradient of some 20°C/hour, lasts 6 hours, after which the free fatty acid contents in raw material is lower than 1 %.

The non-consumed catalyzer (i.e., hypophosphorous acid) is removed with aqueous washing once the reaction mass has cooled down below 80°C, and on sterification and washing completion molecular distillation is made with the reactant mass under the following conditions:
- Temperature 185°C
- Absolute pressure 0,001 mbar
- Spin speed 400 r.p.m.

Tocopherols, sterols and hydrocarbons are distilled under these conditions, with the resulting glycerides and PTM remaining in the distillation residue.

Distilled fraction reaches about 25% of the initial mass, and their tocopherol contents are some 20%.

Molecular distillation residue, composed approximately by 50% PTM, esters, di- and tri-glyceride fatty acids and some small amounts of monoglycerides, has a valuable application as a viscosity regulator in lubricant oils.

Distillates obtained from molecular distillation are composed of the sterolic fraction, high-molecular weight hydrocarbons (squalenes), tocopherols and some sterol-esters resulting from sterification dissolved in a 1:7 methyl alcohol dissolution and sharply cooled down to -20°C. Under these conditions, sterols and hydrocarbons (squalenes) being present are insoluble and are split from the alcohol solution by spin or filtration.

Subsequently, methanol atmospheric distillation is made, with the distillate, under laminar flow condition, remaining in the evaporation concentrate of methanol falling-filim, yielding a tocopherol concentrate higher than 50%.

This concentrate is then subject to a second molecular distillation under the following conditions:
- Temperature 182°C
- Absolute pressure 0,0005 mbar
- Spin speed 550 r.p.m.

Under the above conditions, a light yellow oily distillate is obtained with high viscosity (higher than 6,000 c.p.s. at 20°C), with a large tocopherol content (higher than 70%).

The distillation residue consists of monoglycerides, which in the first molecular distillation distills hydrocarbons, sterols, and esters with a tocopherol content lower than 6%, which indicates a recovery in the tocopherol process up to above the initial 80%.

### PREFERENTIAL PROCEDURES OF THE INVENTION

The process for the extraction and purification of natural-occurring tocopherols and sterols by sterification with PTM put forward by the present invention may be carried out as follows:
Use 500 gr. of sunflower oil raw materials (industrial deodorization) with the following analytic specifications:
   - Free fatty acids 37.5%
   - Monoglycerides 4.8%
   - Hydrocarbons 4.16%
   - Free esters 6.91%
   - Sterol-esters 2.61%
   - Alpha-tocopherol 5.35%
   - Di- and tri-glycerides 35.65%
   - Others not identified 3.1%
Pour the 500 gr. of raw materials in a 1,000-millimeter flask adding it to 32 gr. of PTM and to 10 gr. of 50% hypophosphorous acid.

The temperature is raised up to 60°C while stirring the mixture and, at this point, the vacuum equipment is switched on and temperature is raised up to 120°C while stirring.

Two hours later, the temperature is raised to 140°C and, after two additional hours, to 160°C, with a temperature up to 190°C in the two last hours of the process.

On completion of this time period, the heater is switched off and mixture is cooled down to 60°C.

Then acidity is analyzed by tritation and G.C. with a FID detector, with the following output:
- Fatty acid content 0.8%
- Tocopherol content 5.29%

Subsequently, the UTA molecular distillation equipment is fed with 150-gr/h flow under the conditions above given in the specification, resulting in optimization of 126.2-gr of distillate with a tocopherol content of 20.08%.

The distillate is then dissolved in 875 ml methanol and cooled down to -28°C in a freezer.

Next, the methanol solution is filtered and the methanol is distilled, yielding 62.49 gr. tocopherol concentrate with an alpha- tocopherol content of 50.01 % as a residue.

This residue undergoes a second molecular distillation in the same equipment under the above conditions with a 125-gr/h flow, which yields 35.15 gr. of 72% tocopherol.

In a second procedure, take 500 gr. distillate from soy oil deodorization composed as follows:
- Fatty acids 31.37%
- Free sterols 5.41%
- Sterol-esters 2.11%
- Alpha-tocopherol 1.84%
- Beta + gamma tocopherols 4.21%
- Delta-tocopherol 2.04%

Proceeding in the same way as in the first procedure, add 3 gr. of PTM and 10 gr. of 50% hypophosphorous acid. The first molecular distillation yields 148 gr. with a total 26.4% tocopherol content.

The same volume of methanol as in the first procedure is dissolved and filtration is performed under the same conditions.

Once methanol has been distilled, 74.6 gr. of residue with a 51.2% content of tocopherols is obtained. Then a second molecular distillation under the same conditions in the first procedure is carried out, yielding a distillate of 51.4 gr. with a 72.16% tocopherol content.

## Claims

1. Process for the extraction and purification of natural-occurring tocopherols and sterols by sterification with propane trimethylol (PTM), with the particularity that fatty acid sterification is carried out by using hypophosphorous acid as the catalyzer and PTM as an alcohol. Likewise, the present process is **characterized by** the fact that dilution is conducted with 1:7 methanol, with the spin or filtration being made at a temperature of -30°C. Molecular distillation is performed in order to split esters from fatty acids, PTM, tocopherols, sterols and hydrocarbons, as well as molecular distillation aimed to purify tocopherols under the aforementioned conditions (see text above).

2. Process for the extraction and purification of natural-occurring tocopherols and sterols by sterification with PTM, according to claim 1. (see above), with the particularity that fatty acid sterification is roughly 35 % in weight and is carried out with PTM, with alcohol having 6 carbon atoms, the hydroxyl groups (alcohol) being placed in primary carbons and with a molecular weight of almost 135, with sterification degrees above 95 % and a starting temperature of 120°C and an end one of 190°C. A further peculiarity is the vacuum process (35-40 torr) conducted with the water resulting from sterification being removed *in continuo*, which shifts the reaction balance towards sterification and prevents hydrolysis.

3. Process for the extraction and purification of natural-occurring tocopherols and sterols by sterification with PTM, according to claim 1. (see above), **characterized by** the peculiarity that the catalyzer used is hypophosphorous acid in 50% aqueous solution.

4. Process for the extraction and purification of natural-occurring tocopherols and sterols by sterification with PTM, according to claim 1. (see above), **characterized by** the peculiarity that the reaction yields a temperature gradient of some 20°C/h for 6 hours, after which free fatty acid contents of raw material is found to be lower than 1%.

5. Process for the extraction and purification of natural-occurring tocopherols and sterols by sterification with PTM, according to claim 1. (see above), with the particularity that the non-consumed catalyzer (hypophosphorous acid) is removed by aqueous washing once the reaction mass has been cooled down under 80°C and that, on sterification and washing completion, a molecular distillation of the reactant mass at a temperature of 185°C, a pressure of 0.001 mbar and a spin speed of 400 r.p.m is conducted. As a result and under the above conditions, tocopherols, sterols and hydrocarbons are distilled, with resulting glycerides and PTM esters remaining in the distillation residue. The distillated fraction is roughly 25% of the initial volume with tocopherol content approaching 20%.

6. Process for the extraction and purification of natural-occurring tocopherols and sterols by sterification with PTM, according to claim 5. (see above), **characterized by** the fact that molecular distillation residue consists of approximately 50% PTM esters, di- and tri-glyceride fatty acids and some small amounts of monoglycerides.

7. Process for the extraction and purification of natural-occurring tocopherols and sterols by sterification with PTM, according to claim 1. (see above), with the peculiarity that the distillate resulting from the molecular distillation is composed of a sterolic fraction, high molecular-weight hydrocarbons, tocopherols and some sterol-esters formed in the sterification process. This distillate is dissolved in a 1:7 dilution in methyl alcohol and sharply cooled down to -20°C, which results in insolubilization of presenting sterols and hydrocarbons, which are then split from the alcoholic solution either by spin or filtration.

8. Process for the extraction and purification of natural-occurring tocopherols and sterols by sterification with PTM, according to claim 7. (see above), **characterized by** the fact that atmospheric distillation of methanol is next undertaken with a still, a distillation which, under the condition of laminar flow, forces the distillate to remain in the evaporation concentrate of methanol falling-filim, which, in turn, yields a tocopherol concentrate above 50%.

9. Process for the extraction and purification of natural-occurring tocopherols and sterols by sterification with PTM, according to claims 7. and 8. (see above), with the particularity that the concentrate subsequently undergoes a second molecular distillation under the following conditions: temperature 182°C, absolute pressure 0.0005 mbar and spin speed 550 r.p.m., which yields a light yellow oily distillate with high viscosity (over 6,000 c.p.s. at 20°C) with a tocopherol-content above 70%.

10. Process for the extraction and purification of natural-occurring tocopherols and sterols by sterification with PTM, according to claim 1. (see above), **characterized by** the fact that the distillation residue consists of monoglycerides from the first molecular distillation, as well as of hydrocarbons, sterols and esters with a tocopherol content lower than 6 %, which turns to the initial 80% during the tocopherol process.
